# DEMANDE DE BREVET EUROPEEN

(11) **EP 2 799 421 A1**
(43) Date de publication de la demande: **05.11.2014**
(21) Numéro de dépôt: 14169496.8
(22) Date de dépôt: 12.07.2013
(51) Int. Cl.: C07C 47/58, C07C 47/575, C07C 41/16, C07C 45/51, C07C 51/367, C07C 43/23, C07C 59/64

(54) **Procede de preparation d'alkoxyphenol et d'alkoxyhydroxybenzaldehyde**

(30) Priorité: 26.07.2012 FR 1257275
(62) Demande divisionnaire de: 13735355.3
(71) Demandeur: Rhodia Operations, 93306 Aubervilliers Cedex (FR)
(72) Inventeur: Garel, Laurent, 69003 LYON (FR)
(74) Mandataire: Menville, Laure

(57) **Abrégé**

La présente invention a pour objet un procédé de préparation d'au moins un composé alkoxyhydroxybenzaldéhyde à partir d'au moins un composé hydroxyphénol. Ce procédé se caractérise en ce qu'il comprend :
(i) - la synthèse d'au moins un alkoxyphénol à partir d'au moins un hydroxyphénol en présence d'au moins un agent O-alkylant ;
(ii) - la récupération d'au moins un alkoxyphénol et d'impuretés dont un composé dialkylé du type dialkoxybenzène ;
(iii) - la séparation du dialkoxybenzène de l'alkoxyphénol ;
(iv) - la condensation de l'alkoxyphénol avec l'acide glyoxylique et l'obtention du composé mandélique correspondant ;
(v) - l'oxydation du composé mandélique en alkoxyhydroxybenzaldéhyde correspondant ; et
(vi) - la récupération de l'alkoxyhydroxybenzaldéhyde éventuellement sous forme solide.

## Description

### Domaine technique de l'invention

La présente invention concerne un procédé de préparation d'au moins un composé alkoxyphénol à partir d'au moins un hydroxyphénol.

La présente invention concerne également un procédé de préparation d'au moins un composé alkoxyhydroxybenzaldéhyde à partir d'au moins un composé hydroxyphénol. La présente invention concerne plus spécifiquement un procédé de synthèse de vanilline (VA ou 4-hydroxy-3-méthoxybenzaldéhyde) et/ou d'éthylvanilline (EVA ou 3-éthoxy-4-hydroxybenzaldéhyde. La présente invention concerne en particulier un procédé de préparation de vanilline et/ou d'éthylvanilline à partir d'un précurseur du gaïacol et/ou du guéthol, et en particulier à partir de pyrocatéchol.

### État de la technique

Les alkoxyhydroxybenzaldéhydes sont des produits très importants utilisés d'abord comme arômes et parfums puis ensuite comme produits intermédiaires dans de nombreux domaines tels que par exemple, l'agrochimie, la pharmacie, la cosmétique et autres industries.

En particulier, la vanilline et l'éthylvanilline sont des produits essentiellement à vocation alimentaire. Il est donc important de proposer sur le marché des produits de haute pureté chimique et de bonne qualité gustative tout en assurant un coût de commercialisation le moins élevé possible.

Ainsi, leur procédé de synthèse nécessite d'améliorer les réactions chimiques mises en oeuvre et de réaliser des opérations de séparation et de purification efficaces.

Il a été proposé différents procédés pour la synthèse des aldéhydes aromatiques. Les procédés les plus importants sont basés sur la fonctionnalisation d'un composé phénolique de départ, par exemple, phénol, catéchol, dérivé du catéchol, gaïacol (ou 2-méthoxyphénol), guéthol (ou 2-éthoxyphénol). Intervient, généralement, dans ce type de procédé, le composé phénolique sous une forme salifiée, par exemple, sous forme d'un sel de sodium : un groupe formyle est additionné en position para du groupe hydroxyle présent sur le cycle benzénique, selon différentes méthodes.

Pour préparer le gaïacol, certains procédés consistent à choisir comme matière première l'ortho-nitrochlorobenzène (ONCB).

Ces procédés présentent de nombreux inconvénients par rapport au procédé partant du pyrocatéchol : manipulation de composés toxiques CMR (cancérogènes, mutagènes et reprotoxiques), comme l'o-anisidine ; nombreuses étapes ; réactions exothermiques et dangereuses, comme la nitration ou l'hydrolyse d'un sel de diazonium ; génération d'une quantité importante d'effluents ; formation d'impuretés, comme le 5-chlorogaïacol, qui se retrouvent dans la vanilline sous la forme de 6-chlorovanilline, etc.

Par ailleurs, il existe une voie d'accès à la vanilline totalement différente qui consiste à faire réagir en milieu basique, le gaïacol et l'acide glyoxylique conduisant à l'acide 4-hydroxy-3-méthoxymandélique puis à oxyder le condensat obtenu.

Le fait de synthétiser le gaïacol ou le guéthol à partir de pyrocatéchol est beaucoup plus direct.

Il existe également des procédés de synthèse du para-méthoxyphénol (PMP, ou encore appelé MEHQ pour monométhyléther de l'hydroquinone) à partir d'hydroquinone. Le PMP est utilisé par exemple dans l'industrie comme inhibiteur de polymérisation ou comme anti-oxydant.

### Buts de l'invention

Ces voies d'accès demandent à être améliorées, et en particulier la voie d'accès à partir du pyrocatéchol et/ou de l'hydroquinone. La présente invention a pour but de fabriquer le composé alkoxyphénol de manière industrielle avec un bon rendement, une faible production d'impuretés et/ou leur élimination efficace, et une conversion de l'hydroxyphénol élevée, tout en limitant les coûts d'une telle production. L'invention a pour but en particulier de préparer ce composé alkoxyphénol à partir d'une réaction de O-alkylation mettant en oeuvre un agent O-alkylant. On peut ainsi préparer par exemple le gaïacol à partir de pyrocatéchol, mais il se forme également le vératrole (1,2-diméthoxybenzène) par une réaction de O-méthylation supplémentaire (successive) du gaïacol. Les procédés de préparation d'alkoxyphénol selon l'invention génèrent au moins un composé du type dialkoxybenzène. Ce composé peut être considéré comme une impureté, mais parfois également comme un composé valorisable économiquement. Il est souhaité au vu du bénéfice potentiel de la valorisation économique parallèle de l'alkoxyphénol et des composés du type dialkoxybenzène de pouvoir ajuster la synthèse préférentiellement vers l'un ou l'autre des composés selon la demande du marché, c'est-à-dire de pouvoir former plus ou moins le composé du type dialkoxybenzène par rapport à l'alkoxyphénol, et inversement. Par exemple, le vératrole peut être aujourd'hui valorisé séparément du gaïacol. Ainsi l'invention vise à fournir un procédé de préparation d'au moins un alkoxyphénol à partir d'au moins un hydroxyphénol permettant de moduler ou ajuster la production du rapport dialkoxybenzène/alkoxyphénol.

La présente invention a pour but en particulier de former le composé dialkoxybenzène, comme le 1,2-diméthoxybenzène (vératrole), le 1,2-diéthoxybenzène, le 1,4-diméthoxybenzène (PDMB), ou le 1,4-diéthoxybenzène, lors de la synthèse d'alkoxyphénol, comme le gaïacol, le guéthol, le 4-méthoxyphénol (PMP) et/ou le 4-éthoxyphénol (PEP), selon un ratio satisfaisant les besoins ponctuels du marché, c'est-à-dire selon un ratio dialkoxybenzène/alkoxyphénol compris entre 0,001 et 0,9, en limitant les coûts d'une production industrielle. L'invention a pour but en particulier de fournir un tel procédé tout en maximisant la conversion de l'hydroxyphénol de départ.

Par ailleurs, pour valoriser le composé alkoxyphénol de haute pureté il faut notamment séparer ce dernier du dialkoxybenzène formé. La présente invention a pour but également d'éliminer le composé dialkoxybenzène formé après préparation d'au moins un alkoxyphénol à partir d'au moins un hydroxyphénol. En général un composé dialkoxybenzène est difficile à séparer d'un composé alkoxyphénol. Ceci rend donc difficile l'obtention de l'alkoxyphénol valorisable, c'est-à-dire séparé notamment du dialkoxybenzène formé avec un degré de pureté satisfaisant, tout en maintenant des coûts de production industriels acceptables pour le marché.

Ces problèmes se posent plus spécialement lors de la préparation de vanilline (VA) ou d'éthylvanilline (EVA) à partir de ortho-hydroxyphénol (ou pyrocatéchol, dit « PC » dans la présente invention, ou encore appelé catéchol, ou pyrocatéchine) car l'impureté dite « dialkoxylée » du gaïacol (GA) ou du guéthol (GE), comme en particulier le 1,2-diméthoxybenzène (ou vératrole) et/ou le 1,2-diéthyoxybenzène, est très difficile à séparer de GA et/ou GE. Les composés du type dialkoxybenzène comme le vératrole s'accumulent au cours de la synthèse d'alkoxyhydroxybenzaldéhydes car ils ne réagissent pas avec l'acide glyoxylique. Il est donc important de pouvoir séparer ou éliminer le composé dialkoxybenzène du flux de production d'alkoxyphénoles, et donc également lors de la production d'alkoxyhydroxybenzaldéhydes.

Les mêmes problèmes se posent en termes de performance et qualité de la réaction d'O-alkylation de l'hydroquinone en PMP. On souhaite moduler le rapport PDMB/PMP et éliminer le composé dialkoxylé 1,4-diméthoxybenzène (PDMB) du PMP formé.

Ainsi, l'invention a également pour but de fournir un procédé de préparation à partir d'au moins un hydroxyphénol d'au moins un alkoxyphénol de haute pureté au regard de la présence non souhaitée de composé(s) dialkoxybenzène.

Par ailleurs, la préparation de vanilline et/ou d'éthylvanilline demande à être améliorée dans son ensemble, notamment à partir du précurseur qu'est le pyrocatéchol. C'est un autre but indépendant du procédé de l'invention, de même que la maîtrise de la quantité de vératrole et/ou de 1,2-diéthoxybenzène produite lors d'un procédé de synthèse de vanilline et/ou d'éthylvanilline à partir de pyrocatéchol.

### Résumé de l'invention

La présente invention permet de préparer le composé alkoxyphénol de manière industrielle avec un bon rendement, une faible production d'impuretés et/ou leur élimination efficace, et une conversion de l'hydroxyphénol élevée tout en limitant les coûts d'une production industrielle.

La présente invention permet de moduler ou ajuster le ratio dialkoxybenzène/alkoxyphénol, entre certaines valeurs afin de produire les deux composés selon des quantités satisfaisant les besoins momentanés du marché.

La présente invention permet également d'éliminer le composé dialkoxylé formé lors de la production d'alkoxyphénol.

La synthèse d'alkoxyphénol à partir d'hydroxyphénol est connue que ce soit en phase gazeuse ou en milieu multiphasique par O-alkylation en utilisant un agent O-alkylant. En milieu multiphasique il est connu également que le rapport base/agent alkylant et le rapport agent alkylant/hydroxyphénol permettent d'influencer le ratio dialkoxybenzène/alkoxyphénol. Ces rapports influencent directement les performances réactionnelles, c'est à dire taux de conversion des réactifs, rendements des produits formés, et notamment d'alkoxyphénol et/ou de dialkoxybenzène, et la sélectivité des produits obtenus, en particulier le ratio dialkoxybenzène/alkoxyphénol.

Les inventeurs ont découverts de manière surprenante qu'en milieu comprenant au moins une phase liquide-liquide, et notamment en milieu triphasique, la quantité de solvant organique permettait également de moduler ou d'ajuster le ratio dialkoxybenzène/alkoxyphénol tout en conservant des conditions industrielles très satisfaisantes, voire optimales, en terme de conversion d'hydroxyphénol et de rendements des différents produits. L'art antérieur est à la connaissance des inventeurs muet sur une quelconque influence de la quantité de solvant organique sur le ratio dialkoxybenzène/alkoxyphénol. Il a été constaté dans le cadre de la présente invention que la quantité de solvant organique habituellement utilisée selon l'art antérieur n'a pas d'influence sur le ratio dialkoxybenzène/alkoxyphénol. Mais il a été constaté expérimentalement de manière surprenante par les inventeurs qu'en dessous d'une certaine quantité de solvant organique par mole d'hydroxyphénol dans des conditions de réaction définies, la quantité de solvant organique influence le ratio dialkoxybenzène/alkoxyphénol, de sorte qu'il est possible de moduler ou ajuster ce ratio selon la demande du marché et ainsi valoriser indépendamment les composés dialkoxybenzène et alkoxyphénol, après séparation, tout en minimisant le stock du composé en excès par rapport aux besoins du marché.

L'invention n'aborde pas la séparation et la valorisation du composé dialkoxybenzène.

L'invention concerne ainsi un procédé de préparation d'alkoxyphénol à partir d'hydroxyphénol comprenant une réaction d'O-alkylation d'au moins un hydroxyphénol en au moins un alkoxyphénol, ladite réaction étant mise en oeuvre en utilisant un agent O-alkylant, un solvant aqueux comprenant une base de Bröensted, et un solvant organique, avec un rapport base/agent O-alkylant compris entre 0,5 et 1,5 en moles de base par mole d'agent O-alkylant, un rapport agent O-alkylant/hydroxyphénol compris entre 0,5 et 2 moles d'agent O-alkylant par mole d'hydroxyphénol, et un rapport solvant organique/hydroxyphénol inférieur à 280 mL, de préférence compris entre 10 et 250 mL, et encore de préférence entre 50 et 150 mL de solvant organique par mole d'hydroxyphénol.

Les rapports base/agent O-alkylant, agent O-alkylant/hydroxyphénol, solvant organique/hydroxyphénol selon l'invention, permettent d'ajuster le rendement en composé alkoxyphénol et/ou en composé dialkoxybenzène, et d'ajuster la conversion en hydroxyphénol, et ainsi de les optimiser. On préfère que la quantité de solvant organique utilisée soit inférieure ou égale à la quantité maximale de solvant organique influençant le ratio dialkoxyphénol/alkoxyphénol. La quantité maximale de solvant organique influençant le ratio dialkoxyphénol/alkoxyphénol dépend du solvant lui-même et des conditions de réaction précises mises en oeuvre. On préfère que la quantité de solvant utilisée soit inférieure à 280 mL, de préférence à 250 mL, et encore de préférence à 150 mL, par mole d'hydroxyphénol.

L'invention concerne ainsi un procédé de préparation à partir de pyrocatéchol de gaïacol et/ou de guéthol ou à partir d'hydroquinone de para-méthoxyphénol et/ou de para-éthoxyphénol, par O-méthylation et/ou O-éthylation d'un des groupes hydroxyles du pyrocatéchol ou de l'hydroquinone.

La présente invention concerne également un procédé de préparation d'alkoxyhydroxybenzaldéhyde à partir d'hydroxyphénol comprenant une réaction d'O-alkylation d'au moins un hydroxyphénol en au moins un alkoxyphénol, ladite réaction étant mise en oeuvre en utilisant un agent O-alkylant, un solvant aqueux comprenant une base de Bröensted, et un solvant organique, avec un rapport base/agent O-alkylant compris entre 0,5 et 1,5 en moles de base par mole d'agent O-alkylant, un rapport agent O-alkylant/hydroxyphénol compris entre 0,5 et 2 moles d'agent O-alkylant par mole d'hydroxyphénol, et un rapport solvant organique/hydroxyphénol inférieur à 280 mL, de préférence compris entre 10 et 250 mL, et encore de préférence entre 50 et 150 mL de solvant organique par mole d'hydroxyphénol, et ledit procédé comprenant une réaction apportant une fonction aldéhyde sur l'alkoxyphénol pour obtenir l'alkoxyhydroxybenzaldéhyde correspondant, de préférence par réaction de condensation entre l'alkoxyphénol et l'acide glyoxylique puis oxydation du composé formé.

La présente invention concerne indépendamment un procédé de préparation de vanilline et/ou d'éthylvanilline à partir de pyrocatéchol comprenant une réaction de O-méthylation et/ou d'O-éthylation du pyrocatéchol en gaïacol et/ou en guéthol, ladite réaction étant mise en oeuvre en utilisant un agent O-alkylant, un solvant aqueux comprenant une base de Bröensted, et un solvant organique, avec un rapport base/agent O-alkylant, un rapport agent O-alkylant/hydroxyphénol, et un rapport solvant organique/hydroxyphénol tels que définis ci-dessus, ledit procédé comprenant une réaction apportant une fonction aldéhyde sur le gaïacol et/ou le guéthol pour obtenir la vanilline et/ou d'éthylvanilline, de préférence par réaction de condensation entre le gaïacol et/ou le guéthol et l'acide glyoxylique puis oxydation du produit de condensation. Cet enchaînement spécifique permet de préparer la vanilline et/ou l'éthylvanilline de manière industrielle avec un bon rendement, une faible production d'impuretés et/ou leur élimination efficace, et une conversion du pyrocatéchol élevée.

Sur les figures :
la figure 1 représente un diagramme blocs d'une variante préférée du procédé de préparation d'alkoxyphénol à partir d'hydroxyphénol ;
la figure 2 représente un diagramme blocs d'une variante préférée de la préparation d'alkoxyhydroxybenzaldéhyde à partir d'alkoxyphénol ;
la figure 3 représente un diagramme blocs selon une variante préférée comprenant l'élimination d'un composé dialkoxybenzène préalablement à la réaction de l'alkoxyphénol avec l'acide glyoxylique.
Les traits en pointillés représentent notamment des variantes préférées du procédé de l'invention.

### Description détaillée de l'invention

Le procédé de l'invention s'applique tout particulièrement à la préparation d'alkoxyhydroxyphénols comme le gaïacol et/ou le guéthol, ou le 1,4-méthoxyphénol (para-méthoxyphénol ou PMP) et/ou le 1,4-éthoxyphénol (para-éthoxyphénol ou PEP), mais il convient également pour d'autres alkoxyhydroxyphénols.

Le procédé de l'invention s'applique tout particulièrement à la vanilline et à l'éthylvanilline mais il convient également pour d'autres aldéhydes aromatiques.

Par "hydroxyphénol", on entend au moins un composé de type benzène, substitué ou non, comprenant deux groupes hydroxyles. L'hydroxyphénol peut être substitué par exemple par un ou plusieurs groupes choisi parmi : hydroxyle, alkyle, alkoxy, halogène, nitro, aryle, les groupements comprenant des atomes de carbones pouvant comprendre 1 ou plusieurs hétéroatomes, notamment choisi parmi O, N, et S. Il répond plus particulièrement à la formule (I) suivante : 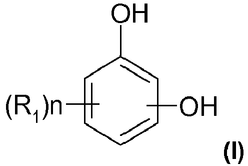 dans ladite formule (I) :
- le ou les groupes R1, identiques ou différents, représentent un atome d'hydrogène, un groupe hydroxyle, un groupe alkyle ou alkoxy,
- n est un nombre de 0 à 3.
La position respective des deux groupes hydroxyles de la formule (I) n'est donc pas fixée. Ils peuvent être en position ortho, méta, ou para.

On peut citer parmi les composés de formule (I) le pyrocatéchol (« PC » ou ortho-hydroxyphénol), la résorcine (« RS » ou méta-hydroxyphénol) et l'hydroquinone (« HQ » ou para-hydroxyphénol), qui sont préférés pour le procédé selon l'invention.

Dans le cadre de l'invention, on entend par "alkyle", une chaîne hydrocarbonée linéaire ou ramifiée ayant de 1 à 12 atomes de carbone et de préférence de 1 à 4 atomes de carbone. Des exemples préférés de groupes alkyles sont les groupes méthyle, éthyle, propyle, isopropyle, tertio-butyle.

Par "alkoxy", on entend un groupe -O-alkyle dans lequel le terme alkyle a la signification donnée ci-dessus. Des exemples préférés de groupes alkoxy sont les groupes méthoxy ou éthoxy.

Par "alkoxyphénol", on entend au moins un composé de type benzène comprenant un groupe hydroxyle et un groupe alkoxy (ou O-alkyl). Il répond plus particulièrement à la formule (II) suivante : 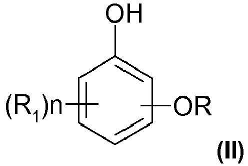 dans ladite formule (II) :
- R représente un groupe alkyle, comprenant de préférence de 1 à 4 atomes de carbone, lié de manière covalente à l'atome d'oxygène ;
- R1, et n sont tels que définis ci-dessus en référence au composé de formule (I).

Les composés alkoxyphénol sont préparés à partir du composé hydroxyphénol correspondant, c'est-à-dire que le groupe R, apporté par un agent d'O-alkylation, remplace un hydrogène d'un des groupes hydroxyle du composé dhydroxyphénol de départ. Cette réaction est appelée réaction d'O-alkylation.

On peut citer en particulier le gaïacol, le guéthol, le para-méthoxyphénol (PMP), et le para-éthoxyphénol (PEP).

Par "alkoxyhydroxybenzaldéhyde", on entend au moins un composé phényl comprenant un groupe hydroxyle, un groupe alkoxy (ou O-alkyl), et un groupe formyle. Il répond plus particulièrement à la formule (III) suivante : 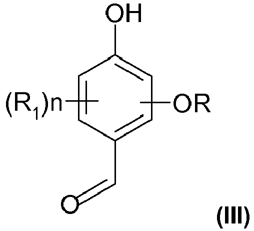 R, R1, et n sont tels que définis ci-dessus en référence au composé de formule (I).

L'alkoxyhydroxybenzaldéhyde mis en oeuvre préférentiellement dans le procédé de l'invention répond à la formule (III) dans laquelle R représente un groupe méthyle ou éthyle et n =0.
Comme exemples plus spécifiques d'alkoxyhydroxybenzaldéhyde, on peut citer notamment :
- la vanilline,
- l'éthylvanilline (3-éthoxy-4-hydroxybenzaldéhyde),
- l'isovanilline (3-hydroxy-4-méthoxybenzaldéhyde),
- l'o-vanilline (2-hydroxy-3-méthoxybenzaldéhyde),
- l'aldéhyde syringique (3,5-diméthoxy-4-hydroxybenzaldéhyde).

### Réaction d'O-alkylation

Selon la présente invention le composé hydroxyphénol peut être transformé en dérivés monoalkoxylés, c'est-à-dire en alkoxyphénol par réaction avec un agent d'O-alkylation.

L'agent d'O-alkylation est choisi avantageusement parmi les composés apportant un groupe alkyl en remplacement de l'atome d'hydrogène lié à l'oxygène du composé hydroxyphénol. Parmi les différents agents d'O-alkylation on peut citer notamment les agents méthylant et les agents éthylant permettant de donner les composés méthoxylés et/ou éthoxylés correspondants.

Selon une variante de l'invention, le pyrocatéchol, le résorcinol ou l'hydroqinone est converti en éther monoalkylique de celui-ci en faisant réagir l'hydroxyphénol avec un chlorure d'alkyle, de préférence ayant de 1 à 4 atomes de carbone.

Selon une variante, le procédé de la présente invention consiste en la préparation à partir de pyrocatéchol de gaïacol et/ou de guéthol ou à partir d'hydroquinone de para-méthoxyphénol et/ou de para-éthoxyphénol, par O-méthylation et/ou O-éthylation d'un des groupes hydroxyles du pyrocatéchol ou de l'hydroquinone.

Avantageusement l'hydroxyphénol est introduit dans le réacteur d'alkylation sous forme d'une solution aqueuse.

Selon une variante préférée de l'invention la réaction est réalisée en milieu triphasique gaz-liquide-liquide. Selon cette variante l'agent d'O-alkylation est présent au moins en phase gazeuse (réaction triphasique G/L/L). L'agent O-alkylant est de préférence choisi parmi un halogénure d'alkyle et de préférence un chlorure ou bromure de méthyle, éthyle, propyle, isopropyle, butyle, isobutyle, butyle tertiaire, amyle, sec-amyle, isoamyle ou d'amyle. La pression de la réaction d'O-alkylation est avantageusement comprise entre 3 et 15 bars, et de préférence comprise entre 5 et 10 bars. L'invention couvre également la présence d'une autre phase, comme par exemple la présence d'un ou plusieurs solides, comme un ou des catalyseurs supportés.

Selon un autre mode de réalisation, l'agent O-alkylant est présent au moins sous forme solide (réaction triphasique S/L/L). L'agent O-alkylant est de préférence choisi parmi un carbonate alkylé ou un sulfate alkylé, et de préférence parmi le diméthyl sulfate, le diméthyl carbonate, le diéthyl sulfate, le diéthyl carbonate, et l'un quelconque de leur mélanges.

Pour la réaction d'O-alkylation, l'agent basique (ou base) est choisi parmi de préférence : un hydroxyde de métal alcalin ou alcalino-terreux, d'ammonium, d'alkylammonium, et de préférence la soude, la potasse, un carbonate ou bicarbonate de métal alcalin, et de préférence un carbonate de sodium ou de potassium, et l'un quelconque de leur mélanges.

De préférence on utilise un rapport agent O-alkylant/hydroxyphénol compris entre 0,5 et 2 moles d'agent O-alkylant par mole d'hydroxyphénol, et encore de préférence comprise entre 0,7 et 1,7.

La quantité d'eau ajoutée peut être assez élevée et peut varier de 0,5 mole d'eau par mole d'hydroxyphénol jusqu'à 30 moles d'eau par mole d'hydroxyphénol, et est de préférence 4 à 30 moles d'eau par mole d'hydroxyphénol. Mais il n'est pas uniquement important d'ajuster la quantité d'eau en fonction de la quantité de d'hydroxyphénol. Avantageusement, l'O-alkylation de l'hydroxyphénol en alkoxyphénol en milieu multiphasique basique présente un ratio solvant organique/eau, en masse/masse, inférieur à 1,5, et de préférence inférieur à 1,2, et encore de préférence allant de 0,05 à 0,8.

Pour la réaction d'O-alkylation, le solvant organique est choisi pour extraire de la phase aqueuse le composé alkoxyphénol au fur et à mesure de sa formation et solubiliser partiellement l'agent d'O-alkylation, et limiter ainsi la formation du composé dialkoxybenzène pour conserver un ratio dialkoxybenzène/alkoxyphénol satisfaisant. Le solvant organique est également choisi pour solubiliser partiellement l'agent d'O-alkylation dans le solvant organique et limiter ainsi la dégradation de l'agent O-alkylant au contact de la base présente en solution aqueuse, notamment pour augmenter le rendement d'O-alkylation en composé alkoxyphénol. Le solvant organique est choisi parmi de préférence : un alcane linéaire, branché ou non, ou cyclique comprenant de 5 à 10 atomes de carbone, par exemple le cyclopentane, cyclohexane, le méthylcyclohexane, cycloheptane, le n-heptane, octane, un alcane halogéné tels que dichloréthylène et dichloropropylène, un alcool aliphatique, comme le méthanol, un solvant organique comprenant de 2 à 10 atomes de carbones et une fonction éther, par exemple le diéthyléther, le diisopropyléther, le méthyltertiobutyléther, un solvant comprenant de 2 à 10 atomes de carbone et une fonction cétone, par exemple l'acétone et la diéthyle cétone, la méthylisobutylcétone, un polyol, comme un glycol ou un poly(éthylèneglycol), un solvant aromatique comprenant de 5 à 12 atomes de carbone, par exemple le toluène, xylènes, benzène, éthylbenzène. On peut utiliser des solvants de qualité alimentaire.

Il est connu par ailleurs comment préparer des composés de type hydroxyphénol. L'hydroxyphénol est préparé par exemple par hydroxylation du phénol. L'hydroxyphénol est avantageusement ajouté sous forme liquide. Ainsi l'hydroxyphénol est de préférence mis en solution en milieu aqueux préalablement à la réaction d'O-alkylation.

Selon une variante préférée, le réacteur dans lequel est réalisée la réaction d'O-alkylation comprend un moyen d'introduction d'une solution d'au moins un hydroxyphénol dans le réacteur, un moyen d'introduction d'au moins un agent O-alkylant dans le réacteur, un moyen d'introduction d'au moins une solution aqueuse basique dans le réacteur, un moyen d'introduction d'au moins un solvant organique dans le réacteur, et au moins un moyen de sortie du mélange réactionnel comprenant notamment l'alkoxyphénol et le dialkoxybenzène formés. Selon une variante, de l'eau est introduite dans le réacteur en sus de l'eau présente dans la base introduite (solution basique), et de l'hydroxyphénol (solution d'hydroxyphénol).

Les conditions de réaction fixées par le procédé de la présente invention permettent très avantageusement d'obtenir en particulier pour la synthèse de gaïacol un ratio vératrole/gaïacol compris en 0,001 et 0,9, et tout en maximisant le taux de conversion du catéchol, de manière industrielle, en limitant les coûts de production, et les impuretés formées.

Les conditions de réaction fixées par le procédé de la présente invention permettent très avantageusement d'obtenir en particulier pour la synthèse de PMP un ratio PDMB/PMP compris en 0,001 et 0,9, et tout en maximisant le taux de conversion d'hydroquinone, de manière industrielle, en limitant les coûts de production, et les impuretés formées.

La réaction donne l'alkoxyphénol avec de très bon rendement.

La température de la réaction d'O-alkylation est de préférence comprise entre 80 et 150°C et de préférence entre 100 et 140°C.

La réaction s'effectue de préférence dans un autoclave.

Le pH de la phase aqueuse lors de la réaction d'O-alkylation est de préférence compris entre 8 et 12.

Le temps de séjour ou le temps de réaction est compris entre 15 minutes et 3 heures et de préférence entre 30 et 90 minutes.

L'invention couvre en particulier la préparation de gaïacol et/ou de guéthol pour leur utilisation dans la synthèse de vanilline et/ou d'éthylvanilline. Il est donc fait référence en particulier à la préparation du Gaïacol (Ga) et/ou du guéthol (Ge), mais l'invention couvre la préparation de tout alkoxyphénol et leurs mélanges, et en particulier la préparation de para-méthoxyphénol (PMP) et/ou para-éthoxyphénol (PEP). L'invention couvre spécifiquement la préparation de gaïacol et/ou de guéthol à partir de pyrocatéchol.

Plus particulièrement, et on peut se référer à la figure 1, le procédé de préparation de l'alkoxyphénol (avantageusement GA et/ou GE ou PMP et/ou PEP) comprend les étapes suivantes :
(a) éventuelle pré-dissolution de l'hydroxyphénol (avantageusement le catéchol (PC) ou l'hydroquinone (HQ)) solide en milieu aqueux ;
(b) O-alkylation d'hydroxyphénol (PC ou HQ) en alkoxyphénol (avantageusement Ga et/ou Ge ou PMP et/ou PEP) en milieu multiphasique (de préférence triphasique G/L/L) basique ;
(c) Acidification du mélange résultant ;
(d) Séparation de l'alkoxyphénol de l'hydroxyphénol, et séparation de l'alkoxyphénol du solvant organique, et récupération au moins du composé alkoxyphénol.

Ces étapes peuvent être mises en oeuvre en continu ou semi-continu ou batch (discontinu).

Par simplicité, il n'est pas fait ici référence au composé dialkoxybenzène formé. Le dialkoxybenzène est formé lorsqu'on synthétise l'alkoxyphénol.

Le composé d'intérêt principal, c'est-à-dire l'alkoxyphénol (avantageusement le Ga et/ou Ge ou PMP et/ou PEP) se retrouve essentiellement en phase organique. L'invention permet de préparer deux qualités principales : notamment le gaïacol et/ ou le guéthol sous forme liquide, et le gaïacol et/ ou le guéthol sous forme solide.

L'acidification (étape (c)) du milieu réactionnel après O-alkylation est de préférence réalisée par ajout d'un acide minéral. On préfère acidifier avec l'acide sulfurique. Cette acidification a pour but de transférer l'alkoxyphénolate formé (avantageusement gaïacolate et/ou guétholate ou para-méthoxyphénolate et/ou para-éthoxyphénolate) présent en phase aqueuse basique dans la phase organique sous la forme alkoxyphénol (gaïacol et/ou guéthol).

L'étape de séparation (étape d) de l'alkoxyphénol de l'hydroxyphénol, et de séparation de l'alkoxyphénol du solvant organique comprend de préférence une décantation du milieu réactionnel acidifié résultant de l'O-alkylation et d'une séparation des phases organique et aqueuse. La phase organique, c'est-à-dire le mélange solvant organique-alkoxyphénol, est distillée pour séparer le composé alkoxyphénol du solvant organique puis déshydratée (élimination de l'eau). Selon une variante préférée, le solvant organique utilisé à l'étape d) peut être identique à celui utilisé à l'étape b) et peut donc être avantageusement recyclé pour être utilisé dans l'étape d'O-alkylation et/ou dans l'étape de traitement de la phase aqueuse.

La phase aqueuse peut être avantageusement traitée pour récupérer l'hydroxyphénol (notamment PC ou HQ), et les éventuels résidus de composé alkoxyphénol (Ga et/ou Ge ou PMP et/ou PEP). Ce traitement comprend de préférence une extraction de la phase aqueuse par un solvant organique, de préférence par le même solvant que celui utilisé pour la réaction d'O-alkylation. La phase organique obtenue suite à cette séparation peut avantageusement être ajoutée à la phase organique obtenue après séparation de la phase de la phase aqueuse.

La séparation selon l'étape (d) du procédé de l'invention comprend avantageusement les étapes suivantes :
(e) décantation des phases aqueuse et organiques et/ou extraction de l'alkoxyphénol (Ga et/ou Ge ou PMP et/ou PEP) résiduel dans la phase aqueuse par un solvant organique ;
(f) distillation de la phase organique comprenant le solvant organique et l'alkoxyphénol (avantageusement Ga et/ou Ge ou PMP et/ou PEP) et recyclage du solvant organique ;
(g) élimination de l'eau (déshydratation) du mélange contenant l'alkoxyphénol (avantageusement Ga et/ou Ge ou PMP et/ou PEP),
(h) distillation du brut réactionnel pour séparer le composé alkoxyphénol, le composé dialkoxybenzène et les goudrons (cf. ci-dessous étape h).

Avantageusement, les étapes e) à g) sont réalisées en continu à l'exception éventuellement de la décantation.

Après l'étape de déshydratation (g) le procédé de l'invention peut comprendre une zone dite discontinue comprenant une étape de distillation de l'alkoxyphénol (avantageusement Ga et/ou Ge ou PMP et/ou PEP) pour le purifier.

Le procédé est donc avantageusement continu, à l'exclusion éventuellement de la distillation de l'alkoxyphénol (avantageusement Ga et/ou Ge ou PMP et/ou PEP).

Le procédé peut comprendre une étape h) de distillation du brut réactionnel, c'est-à-dire la phase organique, dont le solvant organique a été enlevé, est distillée de façon à séparer le composé alkoxyphénol, le composé dialkoxybenzène et les goudrons. Ces derniers peuvent être traités pour récupérer l'hydroxyphénol n'ayant pas réagi lors de la réaction, qui peut ainsi être recyclé. La déshydratation (g) de l'alkoxyphénol (Ga et/ou Ge ou PMP et/ou PEP) brut est donc de préférence suivie d'une distillation (étape h) afin de séparer le composé alkoxyphénol (Ga et/ou Ge ou PMP et/ou PEP) des autres composés organiques présents tels que les éventuels résidus des composés hydroxyphénol (réactif n'ayant pas réagi) et dialkoxybenzène, et des goudrons (dimères, trimères, oligomères, et autres composés organiques de poids moléculaire très élevé).

L'hydroxyphénol (réactif n'ayant pas réagi) est encore de préférence séparé des goudrons par un traitement connu de l'homme de l'art. L'hydroxyphénol est de préférence recyclé pour l'étape d'O-alkylation. L'invention permet de maximiser la conversion de l'hydroxyphénol tout en minimisant les coûts de production.

Le dialkoxybenzène (Vératrole et/ou 1,2-diéthyoxybenzène, ou 1,4-diméthyoxybenzène et/ou 1,4-diéthyoxybenzène) peut être avantageusement valorisé en le traitant par une étape de purification supplémentaire, connue de l'homme de l'art, telle qu'une distillation, ou de lavage(s), ou d'autres techniques de purification.

Conformément à l'invention, la quantité de solvant organique utilisée doit être de préférence inférieure ou égale à la quantité maximale de solvant organique influençant le ratio dialkoxyphénol/alkoxyphénol afin de pouvoir faire varier le ratio dialkoxybenzène/alkoxyphénol et limiter le stock de dialkoxybenzène et d'alkoxyphénol en excès par rapport aux besoins du marché.

L'invention couvre également la préparation d'au moins un alkoxyhydroxybenzaldéhyde à partir d'hydroxyphénol.

### Synthèse d'alkoxyhydroxybenzaldéhyde

Le procédé selon l'invention permet de préparer un alkoxyhydroxybenzaldéhyde par ajout d'une fonction aldéhyde sur le composé alkoxyphénol précité.

Le procédé selon l'invention permet de préparer en particulier la vanilline et/ou l'éthylvanilline à partir du gaïacol et/ou du guéthol. Le mélange gaïacol/guéthol est obtenu selon toutes proportions. Le ratio Ga/Ge dépend des conditions de O-alkylation. Ce ratio massique Ga/Ge peut par exemple varier de 0,1/100 à 100/1. /Le ratio massique vanilline/éthylvanilline varie de préférence de 80/20 à 40/60, et encore de préférence de 80/20 à 45/55. Selon une variante, le ratio molaire vanilline/éthylvanilline est au moins égal à 2. De préférence la proportion de vanilline en poids est comprise entre 65 et 72%, et de préférence entre 60 et 70 %, et la proportion d'éthylvanilline est comprise entre 28 et 35% et de préférence entre 30 et 33 % en poids.

Il est fait référence aux composés alkoxyphénol, gaïacol, guéthol, et aux alkoxyhydroxybenzaldéhydes correspondants indépendamment de leur forme salifiée ou non. Lorsqu'on fait explicitement référence au sel, cela permet de décrire un détail particulier sur cet aspect. L'homme du métier est à même de savoir lorsqu'il est en présence du composé salifié ou non, notamment au vu de la littérature citée et de ses connaissances générales.

La fonction aldéhyde est apportée avantageusement selon deux réactions principales, à savoir une condensation du composé alkoxyphénol avec l'acide glyoxylique et l'oxydation du composé résultant de la réaction de condensation.

Selon un mode de réalisation préféré, le procédé de l'invention comprend en outre les étapes suivantes :
- condensation du composé alkoxyphénol avec l'acide glyoxylique pour obtenir le composé mandélique correspondant ;
- séparation du composé alkoxyphénol de la solution contenant le composé mandélique, avec de préférence le recyclage du composé alkoxyphénol ;
- oxydation du composé mandélique pour obtenir l'alkoxyhydroxybenzaldéhyde ;
- séparation de l'alkoxyhydroxybenzaldéhyde par extraction avec un solvant organique, puis séparation de l'alkoxyhydroxybenzaldéhyde du solvant organique avec de préférence le recyclage du solvant organique d'extraction de l'alkoxyhydroxybenzaldéhyde ; et
- récupération de l'alkoxyhydroxybenzaldéhyde éventuellement sous forme solide.

Lorsque l'on mentionne sans précision « mandélique », « vanilline », ou « éthylvanilliine », on vise le composé para-mandélique, la para-vanilline, ou l'éthyl-para-vanilline respectivement, sauf indication contraire.

Les composés para-mandéliques (formule (IV)), en particulier la préparation de la para-vanilline, ont les formules suivantes :

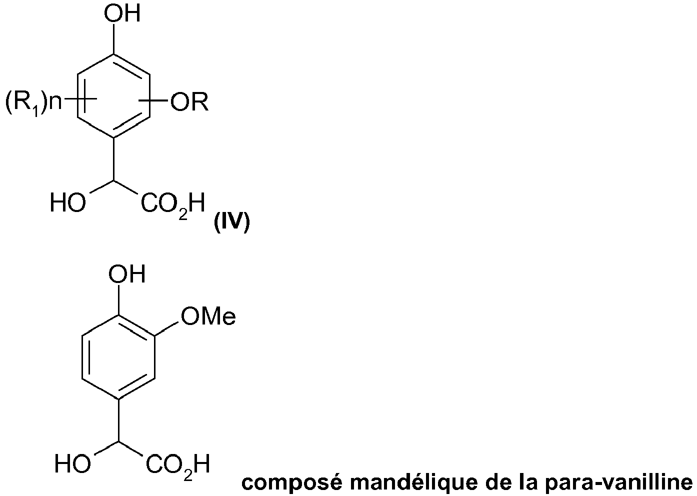

De préférence les étapes et leur enchaînement sont réalisés de manière continue, à l'exception de quelques étapes comme lors de la séparation des composés.

Les réactions de condensation des alkoxyphénols, et en particulier du gaïacol, avec l'acide glyoxylique sont décrites dans la littérature. On citera tout particulièrement les brevets EP 0 578 550, WO 99/65853, et WO 09077383 auxquels on fait référence pour la préparation des composés de l'invention.

L'enchaînement des étapes précédentes peut être réalisé selon les réactions suivantes selon l'exemple du gaïacol :
Condensation du gaïacol et de l'Acide glyoxylique pour former l'acide para-mandélique :

   gaïacol + acide glyoxylique + NaOH → gaïacolate + mandélate (phénate) + H₂O
Acidification pour récupérer le gaïacol non réagi :

   gaïacolate + mandélate (phénate) + H₂SO₄ → gaïacol + mandélate + Na₂SO₄
Oxydation de l'acide para-mandélique pour former la para-Vanilline :

   mandélate + NaOH + O₂ → vanillate + Na₂CO₃ + H₂O
Acidification pour former la vanilline :

   vanillate + Na₂CO₃ + H₂SO₄ → VA + CO₂ + Na₂SO₄

Lors de la séparation du mélange, que ce soit de l'alkoxyphénol, ou de l'alkoxyhydroxybenzaldéhyde ayant conduit à leur formation, on met de préférence en oeuvre un recyclage, appelé également boucle. Ainsi on fait référence dans la présente invention à la « boucle de recyclage 1 » lorsque l'on parle du recyclage de l'alkoxyphénol en excès lors de la réaction de condensation, et de « boucle de recyclage 2 » lorsque l'on fait référence au recyclage du solvant d'extraction de l'alkoxyhydroxybenzaldéhyde, lors des étapes de séparation.

L'alkoxyhydroxybenzaldéhyde une fois séparé peut-être traité pour l'obtenir sous forme purifiée solide, on parle dans la présente invention de « chaine solide ». Ce traitement peut typiquement mettre en oeuvre des étapes de cristallisation, essorage, et séchage, ou écaillage. Parmi ces étapes, certaines, comme l'essorage peuvent ne pas être effectuées de manière continue. Ainsi, de préférence le procédé est continu, à l'exclusion de l'essorage.

La vanilline et/ou l'éthylvanilline ainsi obtenue peut en particulier être utilisée comme arôme dans l'industrie comme par exemple dans l'industrie alimentaire, pharmaceutique ou cosmétique, notamment par exemple pour fabriquer des parfums.

La vanilline et/ou l'éthylvanilline peut également servir d'intermédiaire de synthèse par exemple pour la préparation d'aldéhyde vératrique (3,4 diméthoxybenzaldéhyde) et/ou 3,4-diéthoxybenzaldéhyde.

Dans ce cadre, si le procédé concerne la préparation simultanée de la vanilline et de l'éthylvanilline, on peut soit synthétiser selon un même flux la vanilline et de l'éthylvanilline, à partir d'un flux comprenant du gaïacol et du guéthol, eux-mêmes préparés à partir de catéchol par réaction simultanée avec un agent O-méthylant et un agent O-éthylant ; soit synthétiser séparément du gaïacol et du guéthol puis les mélanger avant l'étape de condensation pour synthétiser selon un même flux la vanilline et de l'éthylvanilline ; soit synthétiser séparément les composés mandéliques par réactions séparées du gaïacol et du guéthol avec l'acide glyoxylique puis mélanger les composés mandéliques correspondants avant l'étape d'oxydation ; soit synthétiser séparément la vanilline et l'éthylvanilline puis les mélanger, par exemple avant la cristallisation.

Ainsi on peut représenter le schéma de la réaction selon la figure 2 qui comprend avantageusement les étapes suivantes :
- succession des étapes a) à d) (et de préférence incluant les étapes e) à h) décrites ci-dessus notamment en référence à la figure 1), pour récupérer au moins un alkoxyphénol ;
- condensation du composé alkoxyphénol (AP) avec l'acide glyoxylique pour obtenir le composé mandélique correspondant, après ajout d'eau, d'acide glyoxylique et d'une base ;
- séparation du composé alkoxyphénol de la solution contenant le composé mandélique correspondant, après ajout d'un solvant organique et d'un acide, avec de préférence le recyclage du composé alkoxyphénol ;
- oxydation du composé mandélique par réaction avec un agent oxydant introduit pour obtenir l'alkoxyhydroxybenzaldéhyde correspondant ;
- séparation de l'alkoxyhydroxybenzaldéhyde par extraction après ajout d'un solvant organique et d'un acide ;
- séparation de l'alkoxyhydroxybenzaldéhyde du solvant organique par distillation, avec de préférence le recyclage du solvant organique ; et
- récupération de l'alkoxyhydroxybenzaldéhyde (AHBA), éventuellement sous forme solide.

### Réaction de condensation

Selon une variante avantageuse, l'alkoxyphénol est mis à réagir avec l'acide glyoxylique en présence d'une base minérale ou d'une base organique, et de préférence NaOH ou KOH, et encore de préférence en présence de soude (NaOH), ou l'hydroxyde d'ammonium. Selon cette variante on obtient un mélange d'alkylphénolate et de dérivé du type mandélate.

La réaction de condensation entre l'alkoxyphénol (sous forme de phénolate) avec l'acide glyoxylique permet la synthèse de l'acide para-mandélique correspondant.

La condensation peut être réalisée dans des réacteurs agités en cascade. Selon une variante, la réaction est réalisée dans un réacteur à écoulement piston, éventuellement comprenant un échangeur thermique. Un tel mode de réalisation est par exemple décrit pour le gaïacol dans la demande WO 09/077383. La réaction de condensation entre l'alkoxyphénol et l'acide glyoxylique peut être réalisée dans l'eau, en présence d'un métal alcalin, ladite réaction étant conduite en réacteur à écoulement piston. Elle peut être mise en oeuvre dans un réacteur tubulaire.

La réaction de condensation peut être avantageusement catalysée par un hydroxyde d'ammonium quaternaire, comme par exemple selon la réaction décrite dans la demande de brevet EP 0 578 550.

La réaction de condensation peut être avantageusement réalisée en présence d'un acide dicarboxylique, comme par exemple selon la réaction décrite dans la demande de brevet WO 99/65853.

L'alkoxyphénol, étant habituellement en excès par rapport à l'acide glyoxylique, la fraction d'alkoxyphénol n'ayant pas réagi est avantageusement récupérée de la boucle de recyclage 1. Cet excès diminue la probabilité de former des composés du type acide dimandélique (c'est-à-dire des composés issus de la condensation de deux molécules d'acides glyoxylique sur une molécule de gaïacol).

Avantageusement, dans un 1^{er} temps, l'alkoxyphénol et la soude réagissent pour former l'alkoxyphénolate de soude. Par exemple pour le gaïacol :

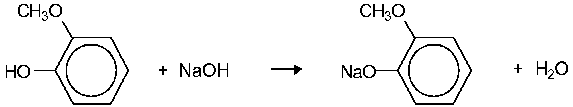

Puis l'alkoxyphénolate réagit avec l'acide glyoxylique pour former le para-mandélate correspondant :

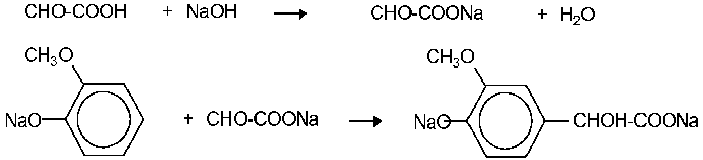

Ces deux réactions de préparation du glyoxylate et du gaïacolate peuvent être mises en oeuvre selon deux étapes séparées. Alternativement l'acide glyoxylique mis en contact directement avec le gaïacolate en présence de la base.

### Solution d'alkoxyphénolate

Selon une variante avantageuse on prépare une solution d'alkoxyphénolate. Cette solution d'alkoxyphénolate est alimentée en l'alkoxyphénol à partir d'une cuve de stockage qui reçoit l'alkoxyphénol recyclé de la boucle de recyclage 1 et l'alkoxyphénol neuf. Cette solution d'alkoxyphénolate est avantageusement introduite dans le réacteur pour la réaction de condensation.

La totalité de l'eau (déminéralisée) et d'une base minérale (par exemple soude) nécessaires à la réaction sont alimentés dans la solution d'alkoxyphénolate.

La température du réacteur de préparation de la solution d'alkoxyphénolate est avantageusement maintenue à la température de la réaction de condensation.

### Boucle de recyclage 1

La boucle de recyclage 1 est la récupération de l'alkoxyphénol n'ayant pas réagi à la réaction de condensation. Ainsi la boucle 1 concerne la séparation du composé alkoxyphénol des composés mandéliques, qui comprend avantageusement les étapes suivantes :
- récupération d'un mélange comprenant le composé mandélique formé sous forme de mandélate et de l'alkoxyphénol n'ayant pas réagi sous forme d'alkoxyphénolate,
- neutralisation de l'alkoxyphénolate en l'alkoxyphénol en maintenant le mandélate correspondant sous forme de base conjuguée (salifiée) ;
- séparation de l'alkoxyphénol du mandélate ;
- introduction du mandélate dans un réacteur d'oxydation ; et
- recyclage de l'alkoxyphénol, n'ayant pas réagi comme réactif, à la réaction de condensation.

Le milieu réactionnel issu de la condensation est avantageusement neutralisé par un acide minéral, et de préférence par l'acide sulfurique, pour transformer l'alkoxyphénolate n'ayant pas réagi en l'alkoxyphénol dans des conditions conservant le mandélate sous forme salifiée.

On peut séparer l'alkoxyphénol n'ayant pas réagi, présent en phase organique, du mandélate formé présent en phase aqueuse.

Selon une variante, on peut extraire l'alkoxyphénol obtenu après neutralisation par extraction par ajout d'un solvant organique qui solubilise l'alkoxyphénol contenu dans la phase aqueuse. On fait référence ici par exemple au procédé décrit pour le gaïacol dans le brevet FR 2 379 501.

Selon un mode de réalisation préféré, l'alkoxyphénol contenu dans la phase aqueuse est extrait par un solvant organique. On fait appel à un solvant organique qui soit inerte par rapport au mandélate. Comme solvants susceptibles d'être utilisés, on peut citer notamment les hydrocarbures aliphatiques, cycloaliphatiques, aromatiques, halogènes ou non ; les alcools ; les éthers, les cétones et les nitriles. On mentionne plus particulièrement comme hydrocarbures aliphatiques, cycloaliphatiques ou aromatiques, l'heptane, le cyclohexane, le méthylcyclohexane, le benzène, le toluène ; comme hydrocarbures aliphatiques, cycloaliphatiques ou aromatiques halogènes, le dichlorométhane, le trichlorométhane, le dichloroéthane, le chlorobenzène, les dichlorobenzènes ; comme alcools, le méthanol, l'éthanol, le propanol, l'isopropanol, le butanol ; comme éthers, le diéthyléther, le diisopropyléther, le méthyltertiobutyléther, comme cétones, l'acétone, la méthyléthylcétone, la méthylisobutylcétone, la diisobutylcétone, la méthylisobutylcétone ; comme nitriles, l'acétonitrile. On peut mettre en oeuvre un mélange desdits solvants. On peut utiliser un solvant de qualité alimentaire.

Le mandélate reste alors en phase aqueuse alors que l'alkoxyphénol passe en phase organique.

L'alkoxyphénol à recycler peut être contaminé par la présence de résidus de solvant organique. La présence de solvant organique dans l'alkoxyphénol conduit à une baisse importante des performances de la réaction de condensation. Il est donc préférable de séparer le solvant d'extraction de l'alkoxyphénol avant sa réintroduction dans la cuve de stockage de l'alkoxyphénol.

Après séparation des phases aqueuse et organique, le mélange alkoxyphénol-phase organique est de préférence distillé pour séparer le solvant organique de l'alkoxyphénol, ainsi que de goudrons généralement présents, afin de recycler le solvant organique. D'autres types de séparation peuvent être envisagés.

La phase aqueuse contenant le mandélate est de préférence traitée pour éliminer le solvant organique résiduel, par exemple par distillation. Le mandélate alimente ensuite la réaction d'oxydation.

Selon une variante, on peut séparer l'alkoxyphénolate n'ayant pas réagi en utilisant des résines échangeuses d'anions basique ou un adsorbant conduisant à la fixation sélective de l'alkoxyphénol et à la récupération d'un flux aqueux comprenant les composés mandéliques sous forme salifiée issus de la réaction de condensation. On peut séparer le flux de l'alkoxyphénol sous forme salifiée (alkoxyphénolate) fixé sur la résine ou l'adsorbant par un traitement de régénération de la résine ou de désorption. De telles méthodes de séparation sont notamment décrites dans les demandes de brevet WO 09/141280 et WO 11/039331.

L'alkoxyphénol n'ayant pas réagi lors de la réaction de condensation est de préférence recyclé à l'alimentation de la solution d'alkoxyphénolate.

### Elimination du dialkoxybenzène (appelé « DAB ») éventuellement présent

Comme il était indiqué plus haut, la réaction de O-alkylation génère également un composé dialkoxybenzène. Dans le procédé de l'invention l'alkoxyphénol obtenu après la réaction de O-alkylation est donc en mélange avec un composé de suralkoxylation (dialkoxylation) du type dialkoxybenzène.

Il a été en particulier découvert par les inventeurs que lorsque le vératrole (1,2-diméthoxybenzène) est formé, notamment lorsque le gaïacol a été préparé à partir de pyrocatéchol, le vératrole s'accumule dans la solution de gaïacol du fait du recyclage mais aussi car il ne réagit pas lors de la réaction de condensation avec l'acide glyoxylique. La concentration en vératrole dans la cuve de stockage du gaïacol (mélange neuf + recyclé) est variable. Comme le vératrole ne réagit pas dans les réactions de condensation selon l'invention, le vératrole a donc tendance à s'accumuler dans la solution de gaïacol et fait donc baisser le titre de gaïacol dans la solution injectée dans le réacteur de condensation. Dans le procédé de l'invention on cherche à limiter le vératrole accumulé dans le gaïacol ou la solution de gaïacolate. Ainsi selon un mode de réalisation préféré de l'invention, on élimine le dialkoxybenzène formé. Selon une variante du procédé de l'invention la concentration massique en vératrole dans la solution comprenant AP avant la réaction de condensation est comprise entre de 0,01 à 25% et de préférence compris entre 0,01 et 10%, et encore de préférence compris entre 0,01 et 5%, et est en général d'environ 2%.

L'invention concerne donc un procédé, indépendamment brevetable, de préparation d'au moins un composé alkoxyhydroxybenzaldéhyde à partir d'au moins un composé hydroxyphénol, ledit procédé étant caractérisé en ce qu'il comprend :
(i) - la synthèse d'au moins un alkoxyphénol à partir d'au moins un hydroxyphénol en présence d'au moins un agent O-alkylant ;
(ii) - la récupération d'au moins un alkoxyphénol et d'impuretés dont un composé dialkylé du type dialkoxybenzène (appelé aussi ici « DAB ») ;
(iii) - la séparation du dialkoxybenzène de l'alkoxyphénol ;
(iv) - la condensation de l'alkoxyphénol avec l'acide glyoxylique et l'obtention du composé mandélique correspondant ;
(v) - l'oxydation du composé mandélique en alkoxyhydroxybenzaldéhyde correspondant ; et
(vi) - la récupération de l'alkoxyhydroxybenzaldéhyde éventuellement sous forme solide.

En particulier l'invention consiste en un procédé de préparation d'au moins un alkoxyhydroxybenzaldéhyde choisi parmi la vanilline (4-hydroxy-3-méthoxybenzaldéhyde), l'éthylvanilline (3-éthoxy-4-hydroxybenzaldéhyde), et l'un quelconque de leurs mélanges, à partir de pyrocatéchol.

Selon une variante préférée, l'étape (i) est mise en oeuvre en milieu triphasique comme décrit dans l'invention, incluant toutes les variantes et modes de réalisation décrits sans aucune limitation.

Selon un mode de réalisation préféré, l'étape (iii) de séparation du dialkoxybenzène de l'alkoxyphénol comprend en outre la préparation d'une solution aqueuse d'alkoxyphénol sous forme salifiée (phénolate) ; et la séparation du dialkoxybenzène résiduel du mélange aqueux comprenant l'alkoxyphénoate.

Selon une variante préférée, illustrée par exemple selon la figure 3 le procédé comprend les étapes suivantes :
- préparation d'une solution d'alkoxyphénol (AP) par exemple en mélange formé d'un alkoxyphénol neuf avec un alkoxyphénol provenant de recyclage, ladite solution d'alkoxyphénol comprenant également une impureté du type dialkoxybenzène (DAB) ;
- préparation d'une solution d'alkoxyphénolate par ajout d'eau et d'au moins une base à la solution d'alkoxyphénol ;
- séparation du dialkoxybenzène de l'alkoxyphénol ;
- valorisation éventuelle du dialkoxybenzène ;
- condensation de l'alkoxyphénol avec l'acide glyoxylique et l'obtention du composé mandélique correspondant, par exemple salifié (mandélate).

Selon une variante, la séparation du dialkoxybenzène de l'alkoxyphénol est réalisée par distillation ou décantation.

Avantageusement, les composés mandéliques sont obtenus sous forme salifiée. Ainsi le procédé de l'invention comprend une étape de neutralisation du composé mandélique salifié en composé mandélique sous forme acide.

Selon une variante, l'hydroxyphénol est le pyrocatéchol, l'alkoxyphénol est le gaïacol obtenu à l'étape (i) avec un agent méthylant, et le dialkoxybenzène est le vératrole, et le procédé comprend également la séparation à l'étape (iii) du pyrocatéchol du mélange gaïacol et vératrole.

Selon une variante, l'hydroxyphénol est le pyrocatéchol, l'alkoxyphénol est le guéthol obtenu à l'étape (i) avec un agent éthylant, et le dialkoxybenzène est le 1,2-diéthoxybenzène, et le procédé comprend également la séparation à l'étape (iii) du pyrocatéchol du mélange guéthol et du 1,2-diéthoxybenzène.

Avantageusement, une partie de l'alkoxyphénol (alkoxyphénolate) est avantageusement dérivée vers une colonne de distillation destinée à limiter la teneur en dialkoxybenzène dans le gaïacol avant la réaction de condensation. Le dialkoxybenzène est distillé en tête sous forme hétéro-azéotrope dialkoxybenzène-eau. On préfère une température en pied de colonne de distillation comprise entre 90 et 120°C. Ce procédé permet par exemple pour le gaïacol de limiter la concentration massique en vératrole à 0,01 % dans le gaïacol (ou gaïacolate) utilisé pour la réaction de condensation.

L'hétéro-azéotrope dialkoxybenzène-eau est ensuite avantageusement séparé par décantation ou un autre moyen de séparation.

Le pied de colonne (comprenant l'alkoxyphénolate) est de préférence dirigé vers l'alimentation du réacteur de condensation pour faire réagir par condensation l'alkoxyphénol (alkoxyphénolate) et l'acide glyoxylique.

### Oxydation des composés mandéliques en alkoxyhydroxybenzaldéhyde

L'étape d'oxydation permet la transformation des composés mandéliques en les alkoxyhydroxybenzaldéhydes désirés. Le composé para-mandélique répond plus particulièrement à la formule (IV) précitée. En particulier, on peut préparer la para-vanilline (ici appelée vanilline) sous forme de vanillate par oxydation de l'acide para-mandélique correspondant.

L'alkoxyhydroxybenzaldéhyde est obtenu par oxydation du composé mandélique correspondant, de préférence en atmosphère oxydante, comme O₂ ou l'air.

Selon une variante, le milieu réactionnel est un milieu aqueux alcalin, de préférence une base minérale et encore de préférence de l'hydroxyde de sodium ou de potassium, pour former le phénate correspondant, et pour capter le CO₂ libéré, sous forme de carbonate.

La réaction peut être conduite en continu ou en discontinu, par exemple en milieu fortement dilué dans l'eau.

La réaction peut être catalysée. Un catalyseur de cette réaction d'oxydation peut être choisi parmi les catalyseurs comprenant au moins un élément métallique choisi parmi le groupe formé par le cuivre, le nickel, le cobalt, le fer, le manganèse, et l'un quelconque de leurs mélanges. A titre d'exemples de composés minéraux ou organiques du cuivre, on peut citer notamment comme composés du cuivre, le bromure cuivreux et cuivrique ; l'iodure cuivreux ; le chlorure cuivreux et cuivrique ; le carbonate cuivrique basique ; le nitrate cuivreux et cuivrique ; le sulfate cuivreux et cuivrique ; le sulfite cuivreux ; l'oxyde cuivreux et cuivrique ; l'hydroxyde cuivrique ; l'acétate cuivreux et cuivrique ; le trifluorométhylsulfonate cuivrique. Comme exemples spécifiques de dérivés du nickel, on peut citer les halogénures de nickel (II), tels que chlorure, bromure ou iodure de nickel (II) ; le sulfate de nickel (II) ; le carbonate de nickel (II) ; les sels d'acides organiques comprenant de 1 à 18 atomes de carbone tels que notamment acétate, propionate ; les complexes de nickel (II) tels que acétylacétonate de nickel (II), dichloro-bis-(triphénylphosphine) de nickel (II), le dibromo-bis(bipyridine) de nickel (II) ; les complexes de nickel (0) tels que le bis-(cycloocta-1 ,5-diène) de nickel (0), le bis-diphénylphosphinoéthane de nickel (0). Comme exemples de composés à base de cobalt, on peut citer notamment les halogénures de cobalt (II) et (III) tels que chlorure, bromure ou iodure de cobalt (II) chlorure, bromure ou iodure de cobalt (III) ; le sulfate de cobalt (II) et de cobalt (III) ; le carbonate de cobalt (II), le carbonate basique de cobalt (II) ; l'orthophosphate de cobalt (II) ; le nitrate de cobalt (II) ; l'oxyde de cobalt (II) et de cobalt (III) ; l'hydroxyde de cobalt (II) et de cobalt (III) ; les sels d'acides organiques comprenant de 1 à 18 atomes de carbone tels que notamment l'acétate de cobalt (II) et de cobalt (III), le propionate de cobalt (II) ; les complexes de cobalt (II) tels que le chlorure d'hexaminecobalt (II) ou (III), le sulfate d'hexaminecobalt (II) ou (III), le chlorure de pentaminecobalt (III), le chlorure de triéthylènediaminecobalt (III). On peut également faire appel à des système catalytiques à base de fer, généralement sous la forme d'oxydes, d'hydroxydes ou de sels tels que le chlorure, le bromure, l'iodure, le fluorure de fer (II) et de fer (III) ; le sulfate de fer (II) et de fer (III) ; le nitrate de fer (II) et de fer (III) ; l'oxyde de fer (II) et de fer La réaction d'oxydation peut être catalysée par exemple par un système catalytique comprenant deux éléments métalliques choisis parmi le groupe formé par le cuivre, le nickel, le cobalt, le fer, le manganèse, et l'un quelconque de leur mélanges. La présente invention couvre en particulier les réactions décrites selon la demande de brevet WO 08/148760.

Dans un 1^{er} temps, le composé mandélate réagit avec la base (de préférence la soude) pour salifier la fonction phénate du composé mandélate. Puis l'oxydation en milieu oxydant (de préférence à l'air) produit le vanillate, et du CO₂ (piégé sous forme de carbonate) :

L'oxydation du para-mandélique en para-vanilline est quasi complète, avec une très bonne sélectivité.

A l'issue de la réaction d'oxydation, on obtient le précurseur de l'alkoxyhydroxybenzaldéhyde (vanilline et/ou d'éthylvanilline) c'est-à-dire avec un groupe hydroxyle sous forme salifiée (ionique), et diverses impuretés, dont des goudrons.

Dans une étape suivante, on effectue l'acidification de l'alkoxyhydroxybenzaldéhyde (vanilline et/ou d'éthylvanilline) dans le milieu réactionnel à l'aide d'un acide fort, par exemple l'acide sulfurique. Il faut ensuite récupérer le produit noble à savoir l'alkoxyhydroxybenzaldéhyde (vanilline et/ou d'éthylvanilline) en présence de goudrons selon un procédé qui soit à la fois respectueux de l'intégrité des produits à séparer tout en étant économique et facile à mettre en oeuvre dans des dispositifs industriels tout particulièrement dans ceux qui fonctionnent en continu. Pour séparer l'alkoxyhydroxybenzaldéhyde (vanilline et/ou d'éthylvanilline) du mélange brut réactionnel, une méthode connue consiste à effectuer son extraction à l'aide d'un solvant organique.

Avantageusement le procédé comprend :
- la séparation de l'alkoxyhydroxybenzaldéhyde du mélange réactionnel par extraction avec un solvant organique ; et
- la récupération et le recyclage du solvant organique utilisé pour l'extraction.

### « Boucle de recyclage 2 »

La boucle de recyclage 2 couvre la transformation de l'hydroxylate d'alkoxybenzaldéhyde (vanillate et/ou d'éthylvanillate) obtenu après la réaction d'oxydation en alkoxyhydroxybenzaldéhyde correspondant, et la récupération et le recyclage du solvant utilisé pour l'extraction de l'alkoxyhydroxybenzaldéhyde (vanilline et/ou d'éthylvanilline) du mélange issu de la réaction d'oxydation. L'invention couvre en particulier la préparation de vanilline et/ou d'éthylvanilline.

Le milieu réactionnel issu de l'oxydation est acidifiée par un milieu acide, de préférence contenant un acide minéral, et par exemple de l'acide sulfurique, ou l'acide chlorhydrique, pour transformer le sel d'alkoxyhydroxybenzaldéhyde en alkoxyhydroxybenzaldéhyde.

Selon un mode de réalisation préféré, l'alkoxyhydroxybenzaldéhyde contenu dans la phase aqueuse est extrait par un solvant organique qui solubilise l'alkoxyhydroxybenzaldéhyde contenu dans la phase aqueuse. On fait appel à un solvant organique qui soit inerte par rapport à l'alkoxyhydroxybenzaldéhyde. Comme solvants susceptibles d'être utilisés, on peut citer notamment les hydrocarbures aliphatiques, cycloaliphatiques, aromatiques, halogènes ou non ; les alcools ; les éthers, les cétones et les nitriles. On mentionne plus particulièrement comme hydrocarbures aliphatiques, cycloaliphatiques ou aromatiques, l'heptane, le cyclohexane, le méthylcyclohexane, le benzène, le toluène ; comme hydrocarbures aliphatiques, cycloaliphatiques ou aromatiques halogènes, le dichlorométhane, le trichlorométhane, le dichloroéthane, le chlorobenzène, les dichlorobenzènes ; comme alcools, le méthanol, l'éthanol, le propanol, l'isopropanol, le butanol ; comme éthers, le diéthyléther, le diisopropyléther, le méthyltertiobutyléther, comme cétones, l'acétone, la méthyléthylcétone, la méthylisobutylcétone, la diisobutylcétone ; comme nitriles, l'acétonitrile. On peut mettre en oeuvre un mélange desdits solvants.

L'opération d'extraction est effectuée à une température qui dépend de la nature du solvant.

Cette étape d'extraction peut être réalisée en même temps que l'acidification. Cependant on préfère que le milieu réactionnel et le solvant organique soient mélangés ensemble, puis que l'acide soit ajouté.

Pour isoler l'alkoxyhydroxybenzaldéhyde (vanilline et/ou d'éthylvanilline) du solvant d'extraction, on peut réaliser une séparation par recristallisation mais de préférence on effectue une distillation dudit mélange permettant d'obtenir par exemple en tête de distillation le solvant d'extraction (s'il est le composé le plus volatil du mélange), et par exemple en pied de distillation, un « alkoxyhydroxybenzaldéhyde brut" (vanilline et/ou d'éthylvanilline « brutes »), à savoir un mélange comprenant essentiellement l'alkoxyhydroxybenzaldéhyde, associé à des impuretés lourdes dénommées "goudrons" et à de faibles quantités d'impuretés légères. On fait référence pour cette étape en particulier au procédé décrit dans la demande de brevet WO 2010/007161.

Le solvant organique utilisé est avantageusement recyclé. Il peut être réintroduit dans le procédé pour extraire l'alkoxyhydroxybenzaldéhyde de la phase aqueuse lors de l'acidification, et/ou utilisé pour récupérer l'alkoxyhydroxybenzaldéhyde des eaux de lavage.

Eventuellement l'alkoxyhydroxybenzaldéhyde peut être traité pour le conditionner sous forme solide. De préférence l'alkoxyhydroxybenzaldéhyde purifié, de préférence par distillation, suivie d'une cristallisation (par utilisation d'un ou plusieurs solvants ou par technique d'écaillage), par exemple selon les étapes suivantes :
- dissolution de l'alkoxyhydroxybenzaldéhyde dans un mélange eau-alcool (par exemple méthanol ou éthanol) ;
- cristallisation sous vide ;
- essorage en discontinu ;
- séchage de l'alkoxyhydroxybenzaldéhyde à une température de 25°C à une température inférieure à la température de fusion de l'alkoxyhydroxybenzaldéhyde, de préférence sous gaz inerte (azote typiquement).

On peut envisager plusieurs variantes de cristallisation : par exemple cristallisation discontinue (batch) par exemple par refroidissement, ou cristallisation continue, par exemple sous vide.
Le produit résultant peut être éventuellement broyé pour obtenir des grains plus fins.

### Exemples

Dans les exemples, on définit le taux de conversion, le rendement et la sélectivité obtenus.

Le taux de conversion ou taux de transformation (TT) correspond au rapport entre le nombre de moles de réactif (hydroxyphénol, par exemple) transformé et le nombre de moles de réactif (hydroxyphénol, par exemple) engagé.

Le rendement réel (RR), exprimé par le rapport entre le nombre de moles de produit formé (alkoxyphénol, par exemple) et le nombre de moles de réactif engagé (hydroxyphénol, par exemple).

La sélectivité ou le rendement par rapport au produit transformé (RT), exprimé par le rapport entre le nombre de moles de produit formé (alkoxyphénol, par exemple) et le nombre de moles de réactif transformé (hydroxyphénol, par exemple).

### O-alkylation d'hydroxyphénol : Exemples 1 à 4 (Hydroquinone - « HQ »)

Dans un réacteur en inox 316L de 150 L muni d'une double enveloppe, d'une agitation mécanique, d'un système réfrigérant et d'une arrivée de gaz inerte (azote), on charge en en discontinu (batch) à 80°C :
- 21,9 kg (0,20 kmol) d'hydroquinone (HQ) cristallisée
- x kg d'eau déminéralisée (cf. tableau ci-dessous)
- 2,6 kg (0,02 kmol) d'une solution aqueuse de soude à 30% en poids
- y kg de méthylisobutylcétone (MIBK) (cf. tableau ci-dessous)
On injecte simultanément dans le réacteur contenant la solution aqueuse ci-dessus en 45 minutes sous 10 bars à la température de 120°C :
- 28,3 kg (0,21 kmol) d'une solution aqueuse de soude à 30% en poids
- 11,1 kg (0,22 kmol) de chlorure de méthyle.
Après 1,5 heure de réaction, le milieu réactionnel est refroidi à 30°C et transféré vers la section "neutralisation". Un échantillon de ce milieu réactionnel est prélevé pour doser par chromatographie en phase liquide les composés présents (p-méthoxyphénol PMP et p-diméthoxybenzène PDMB, et HQ) dans le mélange et déterminer les performances réactionnelles.

| Exemples | 1 | 2 | 3 | 4 |
|---|---|---|---|---|
| Eau (x kg) | 43,8 | 30,7 | 12,1 | 0 |
| MIBK (y kg) | 12,7 | 19,1 | 28,7 | 39,0 |
| MIBK / HQ (mL/mol) | 80 | 120 | 180 | 245 |
| TT(HQ) | 85% | 87% | 89% | 89% |
| RR(PMP)/HQ | 65% | 70% | 71% | 71% |
| RR(PDMB)/HQ | 20% | 18% | 16% | 16% |
| R = dialkoxybenzène/ alkoxyphénol (p/p) | 0,34 | 0,29 | 0,25 | 0,25 |

Remarque : On peut préparer en amont une solution aqueuse d'hydroquinone.

Ces exemples illustrent l'effet de la quantité de solvant organique sur le ratio dialkoxybenzène/ alkoxyphénol et en particulier sur le ratio p-diméthoxybenzène/p-méthoxyphénol. Au-delà d'une certaine quantité de solvant organique, il n'y a pas d'effet de la quantité de solvant organique sur le ratio dialkoxybenzène/ alkoxyphénol. Par contre en dessous d'une certaine quantité de solvant organique, l'invention illustre l'effet sur le ratio dialkoxybenzène/ alkoxyphénol. Ceci est surprenant pour l'homme du métier qui ne s'attendait pas à un tel effet.

Ainsi on peut ajuster la synthèse préférentiellement vers l'un ou l'autre des composés selon la demande du marché, c'est-à-dire de pouvoir former plus ou moins le composé du type dialkoxybenzène par rapport à l'alkoxyphénol, et inversement, en utilisant la quantité de solvant organique. Ainsi l'invention concerne un procédé de préparation d'au moins un alkoxyphénol à partir d'au moins un hydroxyphénol permettant de moduler ou ajuster la production du rapport dialkoxybenzène/alkoxyphénol. De plus cet effet est constaté sur un procédé industriel pour lequel la conversion de l'hydroxyphénol de départ est maximisée.

### O-alkylation d'hydroxyphénol : Exemples 5 à 7 (Pyrocatéchol - « PC »)

Dans un réacteur en inox 316L de 150 L muni d'une double enveloppe, d'une agitation mécanique, d'un système réfrigérant et d'une arrivée de gaz inerte (azote), on charge en discontinu (batch) à 80°C :
- 21,2 kg (0,19 kmol) de pyrocatéchol (PC) cristallisée
- 30,7 kg d'eau déminéralisée
- 2,5 kg (0,019 kmol) d'une solution aqueuse de soude à 30% en poids
- x kg de méthylisobutylcétone (MIBK) (cf. tableau ci-dessous)
On injecte simultanément dans le réacteur contenant la solution aqueuse ci-dessus en 45 minutes sous 10 bars à la température de 120°C :
- 21,8 kg (0,164 kmol) d'une solution aqueuse de soude à 30% en poids
- 8,9 kg (0,176 kmol) de chlorure de méthyle.
Après 1,5 heure de réaction, le milieu réactionnel est refroidi à 30°C et transféré vers la section "neutralisation". Un échantillon de ce milieu réactionnel est prélevé pour doser par chromatographie en phase liquide les composés présents (gaïacol GA et vératrole VE et PC résiduel) dans le mélange et déterminer les performances réactionnelles.

| Exemples | 5 | 6 | 7 |
|---|---|---|---|
| MIBK (x kg) | 12,3 | 18,5 | 27,8 |
| MIBK / PC (mL/mol) | 80 | 120 | 180 |
| TT(PC) | 79% | 79% | 80% |
| RR(GA)/PC | 72,1% | 72,4% | 74,5% |
| RR(VE)/PC | 7,3 % | 6,6 % | 5,4 % |
| R = diméthylé / monométhylé (p/p) | 0,11 | 0,10 | 0,08 |

Remarque : On peut préparer en amont une solution aqueuse de pyrocatéchol.

Ces exemples illustrent l'effet de la quantité de solvant organique sur le ratio dialkoxybenzène/ alkoxyphénol et en particulier sur le ratio vératrole / gaïacol.

Comme pour les exemples 1 à 4, au-delà d'une certaine quantité de solvant organique, il n'y a pas d'effet de la quantité de solvant organique sur le ratio dialkoxybenzène/ alkoxyphénol. Par contre en dessous d'une certaine quantité de solvant organique, l'invention illustre l'effet sur le ratio dialkoxybenzène/ alkoxyphénol. Ceci est surprenant pour l'homme du métier qui ne s'attendait pas à un tel effet.

Ainsi on peut ajuster la synthèse préférentiellement vers l'un ou l'autre des composés selon la demande du marché, c'est-à-dire de pouvoir former plus ou moins le composé du type dialkoxybenzène par rapport à l'alkoxyphénol, et inversement, en utilisant la quantité de solvant organique. Ainsi l'invention concerne un procédé de préparation d'au moins un alkoxyphénol à partir d'au moins un hydroxyphénol permettant de moduler ou ajuster la production du rapport dialkoxybenzène/alkoxyphénol. De plus cet effet est constaté sur un procédé industriel pour lequel la conversion de l'hydroxyphénol de départ est maximisée.

### Condensation

Dans un premier réacteur en inox 316L de 5 L muni d'une double enveloppe, d'une agitation mécanique, d'une électrode de pH, d'un système réfrigérant et d'une arrivée de gaz inerte, on charge en continu :
- 64,8 kg/h d'eau déminéralisée
- 9,3 kg/h (116 mol/h) d'une solution aqueuse de soude à 50% en poids.
- 10,1 kg/h (81,5 mol/h) de gaïacol (neuf et recyclé dont on a enlevé tout ou partie du vératrole).
Ce mélange réactionnel est maintenu à la température de 35°C. Cette préparante est ensuite alimentée dans le premier réacteur d'un système de 3 réacteurs en cascade avec une solution aqueuse d'acide glyoxylique à 50% en poids (6,04 kg/h soit 40,8 mol/h).
Les 3 réacteurs parfaitement agités sont en inox 316L et ont chacun un volume de 80 L ; ils opèrent à 35°C.
Le temps de séjour global est de 2 heures.

A la sortie du dernier réacteur, on prélève un échantillon de ce milieu réactionnel et on dose par chromatographie en phase liquide les composés présents dans le mélange.
Les résultats obtenus sont les suivants :
- conversion du gaïacol (GA) : TT = 47%
- acide 4-hydroxy-3-méthoxy mandélique (APM) : RT(APM)/GA=86%
- acide 2-hydroxy-3-méthoxy mandélique : RT(AOM)/GA=5%
- acide 2-hydroxy-3-méthoxy 1,5-dimandélique : RT(ADM/GA)=9%

Le milieu réactionnel est envoyé vers la boucle de recyclage 1 pour séparer le gaïacol en excès des composés mandéliques.

### Oxydation

Le réacteur d'oxydation en inox 316L équipé d'une agitation autoaspirante de type à cavitation ("cavitator") ou de type Rushton et d'une double enveloppe permettant un refroidissement efficace est alimenté en continu par :
- Le mélange des co-catalyseurs et de la solution aqueuse de mandéliques issue de la boucle 1 soit :
   ○ 77 kg/h de milieu réactionnel issu de la réaction de condensation dans lequel le gaïacol en excès est éliminé (puis recyclé) dans la boucle de recyclage 1. Ce mélange contient de l'ordre de 7,2 kg/h d'acide 4-hydroxy-3-méthoxy mandélique, 0,5 kg/h d'acide 2-hydroxy-3-méthoxy mandélique et 0,9 kg/h d'acide 2-hydroxy-3-méthoxy 1,5-dimandélique.
   ○ 100 g/h d'une solution aqueuse de CoCl₂ et de CuSO₄ en une quantité exprimée en pourcentage molaire de métal par rapport à la somme molaire des acides mandéliques : 0,04% chacun.
- la quantité adéquate d'une solution aqueuse de soude à 50% en poids correspondant au moins à la quantité requise par la stoechiométrie de la réaction d'oxydation (à discuter ensemble).
- La quantité d'oxygène à pression atmosphérique suffisante pour avoir une conversion quasi complète des acides mandéliques. L'oxydant peut être de l'oxygène à pression atmosphérique ou de l'air sous pression.
Cette réaction s'effectue à 80°C. A la sortie du réacteur, on prélève un échantillon de ce milieu réactionnel et on dose les composés présents dans le mélange par chromatographie en phase liquide.
Les résultats obtenus sont les suivants :
- conversion de l'acide 4-hydroxy-3-méthoxy mandélique : TT > 99,5%
- Rendement en vanilline VA : RT(VA)/APM=98%

Le milieu réactionnel est envoyé vers la boucle d'extraction du vanillate de sodium de la phase aqueuse (dite boucle de recyclage 2).

Le présent texte décrit les objets suivants :
Objet 1.- Procédé de préparation d'alkoxyphénol à partir d'hydroxyphénol caractérisé en ce qu'il comprend une réaction d'O-alkylation d'au moins un hydroxyphénol en au moins un alkoxyphénol, ladite réaction étant mise en oeuvre en utilisant un agent O-alkylant, un solvant aqueux comprenant une base de Bröensted, et un solvant organique, avec un rapport base/agent O-alkylant compris entre 0,5 et 1,5 en moles de base par mole d'agent O-alkylant, un rapport agent O-alkylant/hydroxyphénol compris entre 0,5 et 2 moles d'agent O-alkylant par mole d'hydroxyphénol, et un rapport solvant organique/hydroxyphénol inférieur à 280 mL, de préférence compris entre 10 et 250 mL, et encore de préférence entre 50 et 150 mL de solvant organique par mole d'hydroxyphénol.
Objet 2.- Procédé de préparation d'alkoxyhydroxybenzaldéhyde à partir d'hydroxyphénol caractérisé en ce qu'il comprend une réaction d'O-alkylation d'au moins un hydroxyphénol en au moins un alkoxyphénol, ladite réaction étant mise en oeuvre selon l'objet 1, et en ce qu'il comprend une réaction apportant une fonction aldéhyde sur l'alkoxyphénol pour obtenir l'alkoxyhydroxybenzaldéhyde correspondant, de préférence par réaction de condensation entre l'alkoxyphénol et l'acide glyoxylique puis oxydation du composé formé.
Objet 3.- Procédé selon l'objet 2, caractérisé en ce qu'il consiste en un procédé de préparation de vanilline et/ou d'éthylvanilline à partir de pyrocatéchol et caractérisé en ce qu'il comprend une réaction de O-méthylation et/ou d'O-éthylation du pyrocatéchol en gaïacol et/ou en guéthol, ladite réaction étant mise en oeuvre selon l'objet 1, et une réaction apportant une fonction aldéhyde sur le gaïacol et/ou le guéthol pour obtenir la vanilline et/ou d'éthylvanilline, de préférence par réaction de condensation entre le gaïacol et/ou le guéthol et l'acide glyoxylique puis oxydation du produit de condensation.
Objet 4.- Procédé selon l'objet 2 ou 3, caractérisé en ce qu'il comprend au moins les étapes suivantes :
   - condensation du composé alkoxyphénol avec l'acide glyoxylique pour obtenir le composé mandélique correspondant ;
   - séparation du composé alkoxyphénol du composé mandélique, avec de préférence le recyclage du composé alkoxyphénol ;
   - oxydation du composé mandélique pour obtenir l'alkoxyhydroxybenzaldéhyde ;
   - séparation de l'alkoxyhydroxybenzaldéhyde par extraction avec un solvant organique, puis séparation de l'alkoxyhydroxybenzaldéhyde du solvant organique avec de préférence le recyclage du solvant organique ; et
   - récupération de l'alkoxyhydroxybenzaldéhyde, éventuellement sous forme solide.
Objet 5.- Procédé selon l'objet 4, caractérisé en ce que la séparation du composé alkoxyphénol du composé mandélique comprend les étapes suivantes :
   - récupération d'un mélange comprenant le composé mandélique formé sous forme de mandélate et de l'alkoxyphénol n'ayant pas réagi sous forme d'alkoxyphénolate,
   - neutralisation de l'alkoxyphénolate en l'alkoxyphénol en maintenant le mandélate correspondant sous forme salifiée ;
   - séparation de l'alkoxyphénol du mandélate ;
   - introduction du mandélate dans un réacteur d'oxydation ; et
   - recyclage de l'alkoxyphénol, n'ayant pas réagi comme réactif, à la réaction de condensation.
Objet 6.- Procédé selon l'un quelconque des objets 2 à 5, caractérisé en ce qu'il comprend :
   - la séparation de l'alkoxyhydroxybenzaldéhyde du mélange réactionnel par extraction avec un solvant organique ; et
   - la récupération et le recyclage du solvant organique utilisé pour l'extraction.
Objet 7.- Procédé selon l'un quelconque des objets 1 à 6, caractérisé en ce que l'obtention de l'alkoxyphénol comprend les étapes suivantes :
   - O-alkylation d'au moins un hydroxyphénol en alkoxyphénol en milieu multiphasique basique ;
   - Acidification du milieu réactionnel résultant ;
   - Séparation de la phase organique contenant le composé alkoxyphénol de la phase aqueuse contenant la base de bröensted et récupération au moins du composé alkoxyphénol.
Objet 8.- Procédé selon l'un quelconque des objets 1 à 7, caractérisé en ce qu'il consiste en la préparation à partir de pyrocatéchol de gaïacol et/ou de guéthol ou à partir d'hydroquinone de para-méthoxyphénol et/ou de para-éthoxyphénol, par O-méthylation et/ou O-éthylation d'un des groupes hydroxyles du pyrocatéchol ou de l'hydroquinone.
Objet 9.- Procédé selon l'un quelconque des objets 1 à 8, caractérisé en ce que l'O-alkylation de l'hydroxyphénol en alkoxyphénol en milieu multiphasique basique présente un ratio solvant organique/eau, en masse/masse, inférieur à 1,5, et de préférence inférieur à 1,2, et encore de préférence allant de 0,05 à 0,8.
Objet 10.- Procédé de préparation d'au moins un composé alkoxyhydroxybenzaldéhyde à partir d'au moins un composé hydroxyphénol, ledit procédé étant caractérisé en ce qu'il comprend :
   (i) - la synthèse d'au moins un alkoxyphénol à partir d'au moins un hydroxyphénol en présence d'au moins un agent O-alkylant tel que défini à l'un quelconque des objets 1, ou 7 à 9 ;
   (ii) - la récupération d'au moins un alkoxyphénol et d'impuretés dont un composé dialkylé du type dialkoxybenzène ;
   (iii) - la séparation du dialkoxybenzène de l'alkoxyphénol ;
   (iv) - la condensation de l'alkoxyphénol avec l'acide glyoxylique et l'obtention du composé mandélique correspondant ;
   (v) - l'oxydation du composé mandélique en alkoxyhydroxybenzaldéhyde correspondant ; et
   (vi) - la récupération de l'alkoxyhydroxybenzaldéhyde éventuellement sous forme solide.
Objet 11.- Procédé selon l'objet 10, caractérisé en ce qu'il consiste en un procédé de préparation d'au moins un alkoxyhydroxybenzaldéhyde choisi parmi la vanilline (4-hydroxy-3-méthoxybenzaldéhyde) et l'éthylvanilline (3-éthoxy-4-hydroxybenzaldéhyde) à partir de pyrocatéchol.

## Revendications

1. Procédé de préparation d'au moins un composé alkoxyhydroxybenzaldéhyde à partir d'au moins un composé hydroxyphénol, ledit procédé étant **caractérisé en ce qu'**il comprend :
(i) - la synthèse d'au moins un alkoxyphénol à partir d'au moins un hydroxyphénol en présence d'au moins un agent O-alkylant ;
(ii) - la récupération d'au moins un alkoxyphénol et d'impuretés dont un composé dialkylé du type dialkoxybenzène ;
(iii) - la séparation du dialkoxybenzène de l'alkoxyphénol ;
(iv) - la condensation de l'alkoxyphénol avec l'acide glyoxylique et l'obtention du composé mandélique correspondant ;
(v) - l'oxydation du composé mandélique en alkoxyhydroxybenzaldéhyde correspondant ; et
(vi) - la récupération de l'alkoxyhydroxybenzaldéhyde éventuellement sous forme solide.

2. Procédé selon la revendication 1, **caractérisé en ce qu'**il s'agit d'un procédé de préparation d'au moins un alkoxyhydroxybenzaldéhyde choisi parmi la vanilline, l'éthylvanilline, et l'un quelconque de leurs mélanges, à partir de pyrocatéchol.

3. Procédé selon l'une ou l'autre des revendications 1 et 2, l'étape (i) étant mise en oeuvre en milieu triphasique.

4. Procédé selon l'une quelconque des revendications 1 à 3, l'étape (iii) de séparation du dialkoxybenzène de l'alkoxyphénol comprenant la préparation d'une solution aqueuse d'alkoxyphénol sous forme salifiée ; et la séparation du dialkoxybenzène résiduel du mélange aqueux comprenant l'alkoxyphénoate.

5. Procédé selon l'une quelconque des revendications 1 à 4, le procédé comprenant les étapes suivantes :
- préparation d'une solution d'alkoxyphénol, ladite solution d'alkoxyphénol comprenant également une impureté du type dialkoxybenzène ;
- préparation d'une solution d'alkoxyphénolate par ajout d'eau et d'au moins une base à la solution d'alkoxyphénol ;
- séparation du dialkoxybenzène de l'alkoxyphénol ;
- valorisation éventuelle du dialkoxybenzène ;
- condensation de l'alkoxyphénol avec l'acide glyoxylique et l'obtention du composé mandélique correspondant.

6. Procédé selon la revendication 5, **caractérisé en ce que** la solution d'alkoxyphénol est un mélange formé d'un alkoxyphénol neuf avec un alkoxyphénol provenant de recyclage.

7. Procédé selon l'une ou l'autre des revendications 5 et 6, la séparation du dialkoxybenzène de l'alkoxyphénol étant réalisée par distillation ou décantation.

8. Procédé selon l'une quelconque des revendications 1 à 7, **caractérisé en ce que** les composés mandéliques sont obtenus sous forme salifiée, et le procédé comprend en outre une étape de neutralisation du composé mandélique salifié en composé mandélique sous forme acide.

9. Procédé selon l'une quelconque des revendications 1 à 8, **caractérisé en ce que** l'hydroxyphénol est le pyrocatéchol, l'alkoxyphénol est le gaïacol obtenu à l'étape (i) avec un agent méthylant, et le dialkoxybenzène est le vératrole, et le procédé comprend également la séparation à l'étape (iii) du pyrocatéchol du mélange gaïacol et vératrole.

10. Procédé selon la revendication 9, **caractérisé en ce que** la concentration massique en vératrole est à 0,01% dans le gaïacol utilisé pour la réaction de condensation.

11. Procédé selon l'une quelconque des revendications 1 à 8, **caractérisé en ce que** l'hydroxyphénol est le pyrocatéchol, l'alkoxyphénol est le guéthol obtenu à l'étape (i) avec un agent éthylant, et le dialkoxybenzène est le 1,2-diéthoxybenzène, et le procédé comprend également la séparation à l'étape (iii) du pyrocatéchol du mélange guéthol et du 1,2-diéthoxybenzène.

12. Procédé selon l'une quelconque des revendications 1 à 11, **caractérisé en ce qu'**une partie de l'alkoxyphénol est dérivée vers une colonne de distillation ; le dialkoxybenzène est distillé en tête sous forme hétéro-azéotrope dialkoxybenzène-eau ; l'hétéro-azéotrope dialkoxybenzène-eau est ensuite séparé par décantation ou un autre moyen de séparation ; et le pied de colonne est dirigé vers l'alimentation d'un réacteur de condensation pour faire réagir par condensation l'alkoxyphénol et l'acide glyoxylique.

13. Procédé selon la revendication 12, **caractérisé en ce que** la température en pied de colonne de distillation est comprise entre 90 et 120°C.
